(19) **Europäisches Patentamt / European Patent Office / Office européen des brevets**

(11) **EP 3 725 928 B1**

(12) # EUROPEAN PATENT SPECIFICATION

(45) Date of publication and mention
of the grant of the patent:
**06.08.2025  Bulletin 2025/32**

(21) Application number: **19743370.9**

(22) Date of filing: **23.01.2019**

(51) International Patent Classification (IPC):
*D04H 3/007* (2012.01)    *A61F 13/15* (2006.01)
*A61F 13/51* (2006.01)    *A61L 15/24* (2006.01)
*C08F 110/06* (2006.01)    *C08K 5/20* (2006.01)
*C08L 23/06* (2006.01)    *C08L 23/12* (2006.01)
*C08L 23/18* (2025.01)    *D01D 5/098* (2006.01)
*D01F 6/46* (2006.01)    *D04H 3/16* (2006.01)
*D04H 3/147* (2012.01)

(52) Cooperative Patent Classification (CPC):
**D04H 3/147; A61F 13/15; A61F 13/51; A61L 15/24;
C08F 110/06; C08K 5/20; C08L 23/06; C08L 23/12;
C08L 23/18; D01D 5/098; D01F 6/46; D04H 3/007;
A61F 2013/51004**

(86) International application number:
**PCT/JP2019/002122**

(87) International publication number:
**WO 2019/146656 (01.08.2019 Gazette 2019/31)**

(54) **SPUN-BONDED NONWOVEN FABRIC, SANITARY MATERIAL AND METHOD FOR MANUFACTURING SPUN-BONDED NONWOVEN FABRIC**

SPINNVLIESSTOFF, HYGIENEMATERIAL UND VERFAHREN ZUR HERSTELLUNG VON SPINNVLIESSTOFF

NON-TISSÉ FILÉ-LIÉ, MATÉRIAU HYGIÉNIQUE ET PROCÉDÉ DE FABRICATION DE NON-TISSÉ FILÉ-LIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.01.2018  JP 2018009762**

(43) Date of publication of application:
**21.10.2020  Bulletin 2020/43**

(73) Proprietor: **Mitsui Chemicals Asahi Life Materials
Co., Ltd.
Tokyo 104-0028 (JP)**

(72) Inventors:
• **ICHIKAWA, Taiichiro
Sodegaura-shi, Chiba 299-0265 (JP)**

• **SHIMADA, Koichi
Sodegaura-shi, Chiba 299-0265 (JP)**
• **MOTOMURA, Shigeyuki
Sodegaura-shi, Chiba 299-0265 (JP)**
• **MATSUBARA, Akio
Tokyo 105-7117 (JP)**

(74) Representative: **Hoffmann Eitle
Patent- und Rechtsanwälte PartmbB
Arabellastraße 30
81925 München (DE)**

(56) References cited:
| | |
|---|---|
| EP-A1- 2 116 367 | EP-A1- 2 644 763 |
| WO-A1-2017/006972 | WO-A1-2017/006972 |
| JP-A- 2011 214 163 | JP-A- 2017 179 658 |
| JP-A- H06 315 968 | JP-A- S5 943 109 |
| US-A1- 2002 019 490 | |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a spunbonded nonwoven fabric, a hygiene material, and a method for producing a spunbonded nonwoven fabric.

Background Art

In recent years, nonwoven fabrics are widely used in various applications because of their excellent air breathability and flexibility. The representative applications of a nonwoven fabrics include, for example, absorbent materials such as paper diaper and sanitary napkin, hygiene mask, medical gauze, and a base fabric of a plaster. Such a nonwoven fabrics is required to have extensibility in accordance with their applications.

For example, in Japanese National-Phase Publication (JP-A) No. H09-512313, a technique for a coherent extensible nonwoven web exhibiting good elongation properties, which contains a polyethylene and a propylene polymer, as a nonwoven fabric contained in a composite nonwoven fabric, has been proposed.

Further, in WO 2014/050965, a spunbonded nonwoven fabric including a composition including two or more kinds of propylenes having melting points different from each other and a specific fatty acid amide, as a nonwoven fabric having flexibility has been proposed.

Furthermore, in Japanese Patent Application Laid-Open (JP-A) No. 2015-071854, as a spunbonded nonwoven fabric that has favorable stretchability and tactile feeling and is suitable for a hygiene material, a spunbonded non-woven fabric including a thermoplastic polyurethane elastomer containing ethylene bisoleic acid amide and/or crosslinked organic fine particles and having a hardness of from 75 to 85 has been proposed.

In addition, in WO 2017/006972, as a spunbonded nonwoven fabric that has a favorable heat sealing property at a low temperature and favorable drawing processability, a spunbonded nonwoven fabric including a propylene homopolymer having a relatively high melting point, and a polymer selected from the group consisting of a random copolymer of an $\alpha$-olefin having a specified number of carbon atoms and propylene, and a propylene homopolymer having a relatively low melting point, a specific mesopentad fraction, and a racemic pentad fraction, in addition to a polyethylene has been proposed.

SUMMARY OF INVENTION

Technical Problem

For the coherent extensible nonwoven web described in JP-A No. H09-512313, an embodiment in which a second extensible layer is contained as a composite nonwoven fabric is expected, however, in order to be used as a single nonwoven fabric, the coherent extensible nonwoven web may be required to have improved extensibility.

The spunbond nonwoven fabric described in WO 2014/050965 may be required to have improved extensibility.

Since the spunbonded non-woven fabric described in JP-ANo. 2015-071854 uses a thermoplastic polyurethane elastomer, the heat resistance is not sufficient, and therefore, the spunbonded non-woven fabric may be required to have improved processability.

The spunbonded nonwoven fabric described in WO 2017/006972 may be required to have more improved extensibility.

An object of an embodiment of the present invention is to provide a spunbonded nonwoven fabric that is excellent in extensibility and a hygiene material using the spunbonded nonwoven fabric, without using a thermoplastic polyurethane elastomer.

An object of another embodiment of the present invention is to provide a method for producing a spunbonded nonwoven fabric that is excellent in extensibility.

Solution to Problem

Means for solving the above-mentioned problems includes the following embodiments.

A spunbonded nonwoven fabric, including:

a fiber formed of a composition containing:

a propylene homopolymer having a melting point of 140°C or higher;
a polyethylene; and

at least one polymer selected from the group consisting of a polymer shown in (I), and a polymer shown in (II), wherein the fiber has a sea-island structure,

an average length of an island phase is from 1 μm to 100 μm in a cross section taken along a major axis direction of the fiber,
a content of the polyethylene is from 1.0 % by mass to 15.0 % by mass with respect to a total amount of the composition,
a content of the at least one polymer selected from the group consisting of the polymer shown in (I) and the polymer shown in (II) is from 5.0 % by mass to 30.0 % by mass with respect to a total amount of the composition, and
a content of the propylene homopolymer having a melting point of 140°C or higher is from 55.0 % by mass to 90.0 % by mass with respect to a total amount of the composition:

(I) a random copolymer of a propylene with at least one selected from ethylene or α-olefin having from 4 to 20 carbon atoms;
(II) a propylene homopolymer having a melting point of less than 120°C satisfying the following (a) to (f):

(a) [mmmm] = 20 mol% to 60 mol%;
(b)

$$[rrrr]/(1 - [mmmm]) \leq 0.1;$$

(c)

$$[rmrm] > 2.5 \ mol\%;$$

(d)

$$[mm] \times [rr]/[(mr)]^2 \leq 2.0;$$

(e)

$$weight\text{-}average \ molecular \ weight \ (Mw) = 10{,}000 \ to \ 200{,}000;$$

and
(f)

$$molecular \ weight \ distribution \ (Mw/Mn) < 4,$$

wherein, in (a) to (d):

[mmmm] is a mesopentad fraction;
[rrrr] is a racemic pentad fraction;
[rmrm] is a racemic meso racemic mesopentad fraction; and
[mm], [rr] and [mr] are respectively triad fractions thereof.

Advantageous Effects of Invention

According to an embodiment of the present invention, a spunbonded nonwoven fabric that is excellent in extensibility and a hygiene material using the spunbonded nonwoven fabric can be provided.
According to another embodiment of the present invention, a method for producing a spunbonded nonwoven fabric that is excellent in extensibility can be provided.

BRIEF DESCRIPTION OF DRAWINGS

Fig. 1A is an image obtained by observing a cross section of a fiber in a spunbonded nonwoven fabric of Example 1 with a transmission electron microscope.

Fig. 1B is an image obtained by observing a cross section of a fiber in a spunbonded nonwoven fabric of Comparative Example 1 with a transmission electron microscope.

Fig. 2 is a schematic diagram of a closed-system spunbond method.

Fig. 3 is a schematic diagram of a gear drawing device.

DESCRIPTION OF EMBODIMENTS

Hereinafter, embodiments of the present invention (hereinafter also refers to as "embodiment") will be described in detail. However, the invention is not limited to the following embodiments. In the following embodiments, constituent elements (including element steps and the like) are not indispensable unless otherwise expressly provided or except the case in which the constituent elements are apparently indispensable in principle. The same applies to numerical values and ranges of the constituent elements, and the invention is not limited thereby.

In the present specification, the term "step" includes not only a separate step but also a step that cannot be clearly distinguished from other steps as long as the object of the step is achieved.

In the present specification, in a case in which plural kinds of substances corresponding to components exist in the composition, the content of each of the components in the composition refers to the total amount of the plural substances in the composition unless otherwise specified.

Regarding stepwise numerical ranges designated in the present specification, an upper or lower limit set forth in a certain numerical range may be replaced by an upper or lower limit of another stepwise numerical range described. Besides, an upper or lower limit set forth in a certain numerical range of the numerical ranges designated in the specification may be replaced by a value indicated in Examples.

In the present specification, a combination of two or more of the preferable embodiments is a more preferable embodiment.

A spunbonded nonwoven fabric according to the embodiment includes a fiber formed of a composition containing a propylene homopolymer having a melting point of 140°C or higher; a polyethylene; and at least one polymer selected from the group consisting of a polymer shown in (I), and a polymer shown in (II), in which the fiber has a sea-island structure, an average length of an island phase is from 1 $\mu$m to 100 $\mu$m in a cross section taken along a major axis direction of the fiber,

a content of the polyethylene is from 1.0 % by mass to 15.0 % by mass with respect to a total amount of the composition,

a content of the at least one polymer selected from the group consisting of the polymer shown in (I) and the polymer shown in (II) is from 5.0 % by mass to 30.0 % by mass with respect to a total amount of the composition, and

a content of the propylene homopolymer having a melting point of 140°C or higher is from 55.0 % by mass to 90.0 % by mass with respect to a total amount of the composition:

(I) a random copolymer of a propylene with at least one selected from ethylene or $\alpha$-olefin having 4 to 20 carbon atoms;

(II) a propylene homopolymer having a melting point of less than 120°C satisfying the following (a) to (f):

(a)

$$[mmmm] = 20 \text{ mol\% to } 60 \text{ mol\%};$$

(b)

$$[rrrr]/(1 - [mmmm]) \le 0.1;$$

(c)

$$[rmrm] > 2.5 \text{ mol\%};$$

(d)

$$[\text{mm}] \times [\text{rr}]/[(\text{mr})]^2 \leq 2.0;$$

(e)

$$\text{weight-average molecular weight (Mw)} = 10{,}000 \text{ to } 200{,}000;$$

and
(f)

$$\text{molecular weight distribution (Mw/Mn)} < 4,$$

wherein, in (a) to (d):

[mmmm] is a mesopentad fraction;
[rrrr] is a racemic pentad fraction;
[rmrm] is a racemic meso racemic mesopentad fraction; and
[mm], [rr] and [mr] are respectively triad fractions thereof.

In this regard, the expression "at least one polymer selected from the group consisting of a polymer shown in (I) and a polymer shown in (II)" may be generally referred to as "specific polymer".

Further, the fiber as a component of the spunbonded nonwoven fabric of the embodiment is made of a composition containing the above respective components, and therefore, a content of each of the components in the fiber is the same as the content of each of the components in the composition.

The spunbonded nonwoven fabric of the embodiment is formed of a fiber made of a composition containing a propylene homopolymer having a relatively high melting point, a polyethylene, and a specific polymer, in which the fiber has a sea-island structure, and an average length of an island phase is from 1 $\mu$m to 100 $\mu$m in a cross section taken along the major axis direction of the fiber.

The present inventors, et al. have obtained novel findings, that is, by including a fiber having a sea-island structure as described above, and by having an average length of an island phase of from 1 $\mu$m to 100 $\mu$m in a cross section taken along the major axis direction of the fiber, the extensibility of the obtained spunbonded nonwoven fabric is increased. It is presumed that when the fiber has a sea-island structure as described above, the oriented crystallization of polypropylene is inhibited and the extensibility is increased, however, the reason why the extensibility is further increased when the average length of the island phase is within the above range is not clear.

The spunbonded nonwoven fabric of the embodiment is formed of a fiber having a sea-island structure. Further, the average length of an island phase in a sea-island structure of the fiber is from 1 $\mu$m to 100 $\mu$m, and preferably from 3 $\mu$m to 50 $\mu$m.

When the length of an island phase is from 1 $\mu$m to 100 $\mu$m, excellent extensibility can be obtained as described above.

In this regard, the length of an island phase in a sea-island structure of a fiber is measured as follows.

At first, a fiber is taken out from a spunbonded nonwoven fabric, the fiber is embedded in paraffin to prepare a measurement sample. Further, the measurement sample is placed on a microtome such that the blade is parallel to the major axis direction of the fiber, and the measurement sample is sliced along the major axis direction of the fiber. After that, the fiber obtained by slicing the measurement sample is carbon-reinforced, and then the cross section is observed by using a transmission electron microscope (TEM), the length of each of arbitrary 100 island phases is measured, and the average length is determined.

In this regard, as the transmission electron microscope, transmission electron microscope model: H-7650 manufactured by Hitachi High-Technologies Corporation is used. The observation magnification is not particularly limited as long as the length of an island phase can be measured, and is, for example, 8000-fold magnification.

In addition, in a case in which the end of the fiber cannot be observed due to the embossing finish, it is better to measure the length of an island phase from the other end to the boundary of the embossing finish. In a case in which both ends of the fiber cannot be observed due to the embossing finish, it is better to measure the length of an island phase from one boundary of the embossing finish to the other boundary of the embossing finish.

That is, the expression "length of an island phase in a sea-island structure of a fiber" in the present invention includes a case of the length of an island phase from one end of the island phase to the boundary of the embossing finish, and a case of the length of an island phase from one boundary of the embossing finish to the other boundary of the embossing finish.

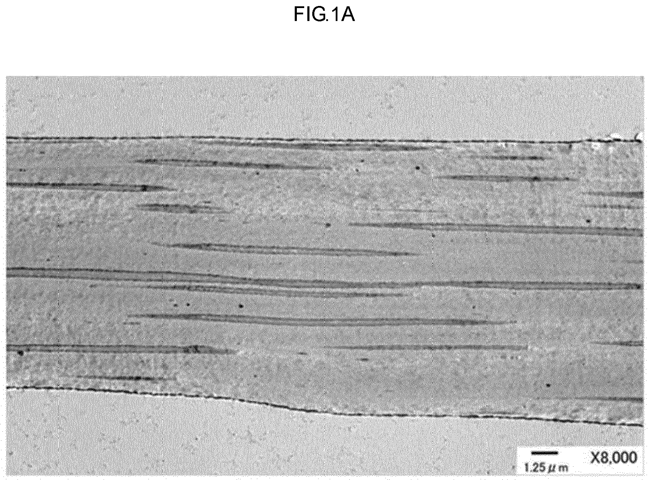

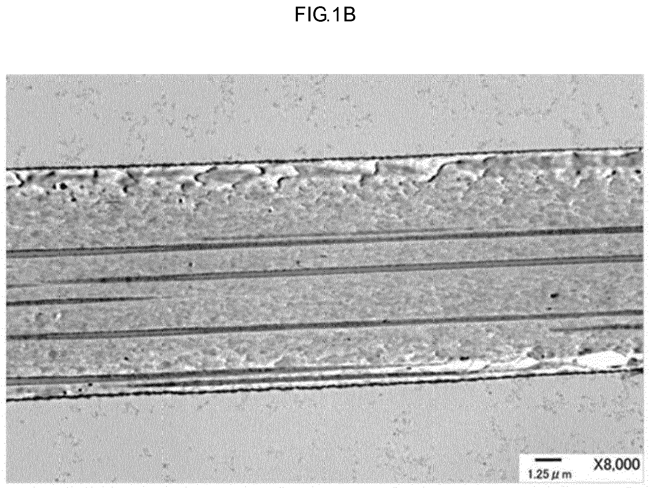

Images in cross sections of fibers observed with a transmission electron microscope are shown in Fig. 1. Fig. 1A is an image in a cross section of a fiber in a spunbonded nonwoven fabric of Example 1, and Fig. 1B is an image in a cross section of a fiber in a spunbonded nonwoven fabric of Comparative Example 1.

It can be confirmed appropriately by a known method that each of the above-described components is contained in the composition as a component of the spunbonded nonwoven fabric of the embodiment or in the fiber made of the composition.

In this regard, the mesopentad fraction [mmmm], the racemic pentad fraction [rrrr], the racemic meso racemic mesopentad fraction [rmrm], and the triad fractions [mm], [rr] and [mr] of a propylene homopolymer having a melting point of less than 120°C in the polymer shown in (II) can be calculated in accordance with a method proposed in "Macromolecules, 6,925 (1973)" by A. Zambelli, et al. as will be described in detail below.

In the composition in the embodiment, the content of the polyethylene is from 1.0% by mass to 15.0% by mass, preferably from 3.0% by mass to 12.0% by mass, and more preferably from 6.0% by mass to 10.0% by mass, with respect to the total amount of the composition.

When the content of the polyethylene is in the above range, the extensibility of the spunbonded nonwoven fabric to be obtained can be improved.

Further, from the viewpoint of improving the strength of a spunbonded nonwoven fabric, it is preferable that the density of the polyethylene is in a range of from 0.941 g/cm$^3$ to 0.970 g/cm$^3$ from the viewpoint of further improving the extensibility and flexibility of the spunbonded nonwoven fabric to be obtained.

When the density of the polyethylene is in the above range, the strength of the spunbonded nonwoven fabric to be obtained can be improved.

In the composition in the embodiment, the content of the specific polymer is from 5.0% by mass to 30.0% by mass, preferably from 10.0% by mass to 30.0% by mass, and more preferably from 15.0% by mass to 25.0% by mass, with respect to the total amount of the composition.

When the content of the specific polymer is in the above range, the extensibility of the spunbonded nonwoven fabric to be obtained can be improved.

From the viewpoint of further improving the extensibility of the spunbonded nonwoven fabric to be obtained, the specific polymer in the embodiment is preferably (I) a random copolymer containing at least a component unit derived from propylene and a component unit derived from ethylene, or (II) a propylene homopolymer having a melting point of less than 120°C and satisfying (a) to (f). From the similar point of view, the specific polymer is more preferably a random copolymer containing at least a component unit derived from propylene and a component unit derived from ethylene, and is still more preferably a random copolymer containing only a component unit derived from propylene and a component unit derived from ethylene.

In the composition in the embodiment, the content of the propylene homopolymer having a melting point of 140°C or higher is from 55.0% by mass to 90.0% by mass, preferably from 60.0% by mass to 85.0% by mass, and more preferably from 65.0% by mass to 80.0% by mass, with respect to the total amount of the composition.

When the content of the propylene homopolymer having a melting point of 140°C or higher is in the above range, the extensibility of the spunbonded nonwoven fabric to be obtained can be improved, and further the strength properties of the spunbonded nonwoven fabric are maintained in a favorable range, and a flexible nonwoven fabric having a low basis weight can be easily obtained.

The composition in the embodiment preferably contains a fatty acid amide having from 15 to 22 carbon atoms. When the composition contains a fatty acid amide having from 15 to 22 carbon atoms, the fatty acid amide having from 15 to 22 carbon atoms is adsorbed onto a fiber surface of the spunbonded nonwoven fabric formed of the composition, and the fiber surface is modified. As a result, the extensibility and flexibility of the spunbonded nonwoven fabric are further improved.

The content of the fatty acid amide having from 15 to 22 carbon atoms is preferably from 0.1% by mass to 5.0% by mass, more preferably from 0.1% by mass to 3.0% by mass, and still more preferably from 0.1% by mass to 1.0% by mass, with respect to the total amount of the composition.

[Embodiment and Physical Properties of Spunbonded Nonwoven Fabric]

Hereinafter, preferable embodiments and physical properties of the spunbonded nonwoven fabric of the embodiment will be described.

(Value Obtained by Dividing 5% Strength by Basis Weight of Nonwoven Fabric and Integrated Value of Stress Integral Value at 50% Drawing)

In the spunbonded nonwoven fabric of the embodiment, it is preferable that the value obtained by dividing the 5% strength by the basis weight of the nonwoven fabric is 0.2 N/25 mm/(g/m$^2$) or more, and further, the integrated value of stress integral value at the time of 50% drawing is 70 N/(g/m$^2$) or less.

The value obtained by dividing the 5% strength by the basis weight of the nonwoven fabric refers to a value obtained by dividing the MD 5% strength that is a tensile load when the nonwoven fabric is in a state drawn 5% from the start state of a tensile test by the basis weight that is a weight per unit area of the nonwoven fabric, by performing the tensile test in accordance with 6.12.1 [method A] of JIS L 1906, in a machine direction (that is, MD direction) during the production of the nonwoven fabric. In addition, the stress integral value at the time of 50% drawing refers to a value obtained by dividing the integrated value of stress integral value from the start state of a tensile test to the state drawn 50% by the basis weight that is a weight per unit area of the nonwoven fabric, by performing the tensile test in accordance with 6.12.1 [method A] of JIS L 1906.

The spunbonded nonwoven fabric having the preferable physical properties can be obtained by using an open-system spunbond method described later.

The basis weight of the spunbonded nonwoven fabric of the embodiment is not particularly limited.

From the viewpoint of achieving both of the flexibility and the strength, in general, the spunbonded nonwoven fabric of the embodiment has a basis weight of preferably 30 g/m$^2$ or less, more preferably 28 g/m$^2$ or less, still more preferably 25 g/m$^2$ or less, and most preferably in a range of from 5 g/m$^2$ to 20 g/m$^2$. In a case in which the spunbonded nonwoven fabric of the embodiment is applied to a hygiene material described later, or the like, the basis weight of the spunbonded nonwoven fabric is preferably in a range of from 5 g/m$^2$ to 19 g/m$^2$.

The basis weight can be measured by a method used in Examples described later.

(Fiber Diameter)

In general, the fiber as a component of the spunbonded nonwoven fabric of the embodiment has a fiber diameter of preferably 50 $\mu$m or less, more preferably 40 $\mu$m or less, still more preferably 30 $\mu$m or less, and most preferably 20 $\mu$m or less. The smaller the fiber diameter is, the more excellent in the flexibility the nonwoven fabric is. From the viewpoint of the handling ability, the production suitability, and the suppression of fluffing of the obtained nonwoven fabric, the fiber diameter is preferably 10 $\mu$m or more.

(Heat Sealability)

As one of the physical properties of a spunbonded nonwoven fabric of the embodiment, heat sealability can be mentioned.

As to the heat sealability of the spunbonded nonwoven fabric, in a case in which two sheets of nonwoven fabrics are superposed on each other and the superposed nonwoven fabrics are heat sealed with a heat-seal testing machine, the heat sealing can be performed preferably at a temperature of 180°C or less, and can be performed more preferably at a temperature of 160°C or less.

Further, it is preferable to suppress burning, that is, discoloration due to heating, in a case in which the heat sealing is performed under the conditions described above.

By confirming the tensile peel strength (also referred to as heat seal strength) of the two sheets of nonwoven fabrics to which heat sealing has been performed, the presence or absence of the heat sealability may be evaluated. For example, the heat seal strength may be appropriately determined in accordance with the intended use of the spunbonded nonwoven fabric, and in general, the heat seal strength is preferably 0.05 N/20 mm or more, and more preferably 0.1 N/20 mm or more.

In addition, the presence or absence of the heat sealability can be confirmed by, for example, a method used in Examples described later.

(Emboss Remaining rate)

As one of the physical properties of a spunbonded nonwoven fabric of the embodiment, an emboss remaining rate can be mentioned.

The emboss remaining rate after drawing processing of the spunbonded nonwoven fabric is preferably 40% or more, more preferably 50% or more, and still more preferably 70% or more.

When the emboss remaining rate after drawing processing is 40% or more, the tactile feeling of the spunbonded nonwoven fabric becomes more favorable.

The emboss remaining rate can be measured by a method used in Examples described later.

(Flexibility)

As one of the physical properties of a spunbonded nonwoven fabric of the embodiment, flexibility can be mentioned.

The flexibility of the spunbonded nonwoven fabric gives large influence on the sense of use of the nonwoven fabric. Examples of the flexibility include flexibility according to sensory evaluation by touch (for example, flexibility in a method described in detail in Examples), and bending resistance. The flexibility can be measured in accordance with 8.21.1 [Method A (45° cantilever method)] of JIS L 1096: 2010.

(Maximum Elongation and Maximum Strength)

As one of the preferable physical properties of a spunbonded nonwoven fabric of the embodiment, maximum elongation and maximum strength can be mentioned.

In the spunbonded nonwoven fabric of the embodiment, the maximum elongation in at least one direction is preferably 70% or more, more preferably 100% or more, and still more preferably 140% or more.

Further, in the spunbonded nonwoven fabric of the embodiment, the maximum strength in at least one direction is preferably 10 N/50 nn or more, more preferably 15 N/50 nn or more, and still more preferably 20 N/50 nn or more.

In addition, a spunbonded nonwoven fabric having a nature of almost no elastic recovery is preferable.

As will be described in detail in Examples, the maximum elongation and maximum strength of the spunbonded nonwoven fabric can be measured in accordance with 6.12.1 [Method A] of JIS L 1906.

The spunbonded nonwoven fabric of the embodiment can be produced by a routine procedure using one kind or two or more kinds of the compositions described in detail below.

[Each Component contained in Composition]

The composition for constituting a spunbonded nonwoven fabric of the embodiment includes, as described above, a propylene homopolymer having a melting point of 140°C or higher (hereinafter also refers to as "specific propylene"); a polyethylene; and at least one polymer (specific polymer) selected from the group consisting of the polymer shown in (I) and the polymer shown in (II).

Hereinafter, each component contained in the composition will be explained in detail.

From the viewpoint of effectively achieving the object of the embodiment, the total content of the specific polypropylene and specific polymer in the total mass of the composition is preferably 80% by mass or more, more preferably 90% by mass or more, and still more preferably 92% by mass or more.

[Propylene Homopolymer Having Melting Point of 140°C or Higher (Specific Polypropylene)]

A propylene homopolymer having a melting point of 140°C or higher contains only a component unit derived from propylene, and is a polymer having a melting point of 140°C or higher.

The melting point of the propylene homopolymer is preferably 150°C or higher. The upper limit of the melting point of the propylene homopolymer is, for example, 166°C.

The specific polypropylene is a crystalline resin that is produced or available on the market under the name of polypropylene, and as the specific polypropylene, any crystalline resin can be used as long as the crystalline resin has a melting point (Tm) of 140°C or higher. As the product available on the market, for example, a homopolymer of propylene, which has a melting point of 155°C or higher and preferably has a melting point in a range of from 157°C to 166°C, can be mentioned.

In this regard, the melting point of the specific polypropylene is indicated by the definition similar to the definition for the melting point of a polymer (I) described later, and can be measured by a method similar to the method for the melting point of a polymer (I) described later.

As to the specific polypropylene, the melt flow rate (MFR: ASTM D 1238, under measurement conditions of a temperature of 230°C and a load of 2160 g) is not particularly limited as long as the specific polypropylene can be melt spun. In general, the melt flow rate of the specific polypropylene is in a range of from 1 g/10 min to 1000 g/10 min, preferably from 5 g/10 min to 500 g/10 min, and still more preferably from 10 g/10 min to 100 g/10 min.

Only one kind of specific polypropylene may be used in a composition, or two or more kinds of specific polypropylenes, which are different from each other in the melting point, the molecular weight, the crystal structures and the like, may be used in a composition.

A preferable content of the specific polypropylene with respect to the total amount of the composition is as described above.

[Polyethylene]

A polyethylene is not particularly limited as long as the polyethylene contains a component unit derived from ethylene, and specifically, as the polyethylene, an ethylene homopolymer such as a high pressure low density polyethylene, a linear low density polyethylene (so-called LLDPE), or a high density polyethylene (so-called HDPE) can be mentioned.

In particular, as the polyethylene, as described above, a high density polyethylene having a density in a range of from 0.941 g/cm$^3$ to 0.970 g/cm$^3$ is preferable from the viewpoint of further improving the extensibility, the flexibility, and the breaking strength.

When a film is formed of a polyethylene, as the polyethylene, a polyethylene of which the number of the particles each having a diameter of 0.2 mm or less contained in the film is 25 pieces/g or less is preferable.

Further, when a film is formed of a polyethylene, as the polyethylene, a polyethylene of which the number of the particles each having a diameter of 2 mm or less contained in the film is 10 pieces/g or less is preferable.

Only one kind of polyethylene may be used in a composition, or two or more kinds of polyethylenes, which are different from each other in the melting point, the molecular weight, the crystal structure and the like, may be used in a composition.

A preferable content of the polyethylene with respect to the total amount of the composition is as described above.

[Specific Polymer]

In the embodiment, by using a composition containing a specific polymer, the obtained spunbonded nonwoven fabric can obtain extremely excellent drawing processability while maintaining favorable flexibility and extensibility.

[Polymer Shown in (I): Random Copolymer of Propylene with at Least One Selected from Ethylene or α-Olefin Having from 4 to 20 Carbon Atoms]

A polymer shown in (I) (hereinafter, also referred to as "polymer (I)") is a random copolymer containing a component unit derived from propylene, and a component unit derived from at least one olefin selected from ethylene or an α-olefin having from 4 to 20 carbon atoms.

When the polymer (I) is a random copolymer, the polymer (I) is preferable from the viewpoint of improving the flexibility without generating sticky feeling in the obtained spunbonded nonwoven fabric.

The polymer (I) is not particularly limited as long as the polymer (I) is a random copolymer containing the above-described component units.

As the component unit that can be copolymerized with propylene, a component unit derived from ethylene, a component unit derived from an α-olefin having from 4 to 20 carbon atoms such as 1-butene, 1-hexene, 1-octene, or 4-methyl-1-pentene, or the like can be mentioned.

Among them, a component unit derived from ethylene, and a component unit derived from an α-olefin having from 4 to 8 carbon atoms are preferable.

The component unit derived from an α-olefin contained in a polymer (I) may be only one kind of component unit, or may also be two or more kinds of component units.

Specifically, preferable examples of the polymer (I) include a propylene/1-butene random copolymer, a propylene/ethylene random copolymer, and a propylene/ethylene/1-butene random copolymer.

From the viewpoint of effectively achieving the object of the embodiment, the total proportion of a component unit derived from propylene and a component unit derived from the above-described olefin other than propylene such as ethylene to the whole component units contained in the polymer (I) is preferably 80 mol% or more, more preferably 85 mol% or more, and still more preferably 90 mol% or more.

The polymer (I) has a melting point of preferably 100°C or higher, more preferably 130°C or higher, and still more preferably 140°C or higher.

The melting point of the polymer (I) is defined as the peak top of a peak observed on the highest temperature side of a melting endothermic curve that has been obtained with the use of a differential scanning calorimeter (DSC) by keeping the polymer (I) at -40°C for 5 minutes under a nitrogen atmosphere, and then raising the temperature at 10°C/min.

Specifically, the melting point can be determined as the peak top of a peak observed on the highest temperature side of a melting endothermic curve that has been obtained with the use of a differential scanning calorimeter (DSC-7, manufactured by Perkin Elmer) by keeping 5 mg of a sample at -40°C for 5 minutes under a nitrogen atmosphere, and then raising the temperature at 10°C/min.

The degree of crystallinity of the polymer (I) is preferably 15% or less, more preferably 10% or less, and still more preferably 8% or less.

The degree of crystallinity of the polymer (I) can be calculated from a heat of fusion curve derived from the melting of the main component in a melting endothermic curve that has been obtained with the use of a differential scanning calorimeter (DSC) by keeping the polymer (I) at -40°C for 5 minutes under a nitrogen atmosphere, and then raising the temperature at 10°C/min.

Specifically, the degree of crystallinity can be calculated from a heat of fusion curve derived from the melting of the main component in a melting endothermic curve that has been obtained with the use of a differential scanning calorimeter (DSC-7, manufactured by Perkin Elmer) by keeping 5 mg of a sample at -40°C for 5 minutes under a nitrogen atmosphere, and then raising the temperature at 10°C/min, by using the following Formula.

$$\text{Degree of crystallinity} = \Delta H / \Delta H0 \times 100\ (\%)$$

In the Formula, $\Delta H$ represents a heat of fusion (J/g) determined from a heat of fusion curve derived from the melting of the main component of an $\alpha$-olefin copolymer containing ethylene and propylene, and $\Delta H0$ represents a heat of fusion (J/g) of a perfect crystal of the main component. That is, in a case in which the main component is ethylene, $\Delta H0$ is 293 J/g, and in a case in which the main component is propylene, $\Delta H0$ is 210 J/g.

The tensile elastic modulus of the polymer (I), which is measured by a method in accordance with JIS K 7161: 2011, is preferably 100 MPa or less, more preferably 40 MPa or less, and still more preferably 25 MPa or less.

From the viewpoint of obtaining the favorable spinnability and the excellent drawing processability, in general, the melt flow rate (MFR, under measurement conditions of a temperature of 230°C and a load of 2160 g) of the polymer (I), which is measured by a method in accordance with ASTM D 1238, is preferably in a range of from 1 g/10 min to 100 g/10 min, more preferably in a range of from 5 g/10 min to 100 g/10 min, and still more preferably in a range of from 30 g/10 min to 70 g/10 min.

The ratio of a weight-average molecular weight (Mw) to a number-average molecular weight (Mn) of the polymer (I): Mw/Mn (molecular weight distribution) is generally from 1.5 to 5.0. From the viewpoint of obtaining a conjugated fiber having more favorable spinnability and particularly excellent fiber strength, the molecular weight distribution (Mw/Mn) of the polymer (I) is further preferably from 1.5 to 3.0.

In this regard, the expression "favorable spinnability" means that thread breakage is not generated during the discharging of the polymer (I) through a spinning nozzle and during the drawing, and further that fusion of filaments is not generated.

Mw and Mn of the polymer (I) can be measured by a known method by gel permeation chromatography (GPC).

In this regard, Mw and Mn of the polymer (I) can be measured by a method similar to the method for the Mw and Mn of the polymer (II) described later.

[Polymer Shown in (II): Propylene Homopolymer Satisfying Following (a) To (f)]

A polymer shown in (II) (hereinafter, also referred to as "polymer (II)") is a polymer satisfying the following (a) to (f).

At first, requirements (a) to (f) will be described.

(a)

$$[\text{mmmm}] = 20\ \text{mol\%}\ \text{to}\ 60\ \text{mol\%}:$$

In a case in which the mesopentad fraction [mmmm] of the polymer (II) is 20 mol% or more, the occurrence of stickiness is suppressed, and in a case in which the mesopentad fraction is 60 mol% or less, the degree of crystallinity does not become too high, so that the elastic recoverability is good. The mesopentad fraction [mmmm] is preferably from 30 mol% to 50 mol%, and more preferably from 40 mol% to 50 mol%.

The mesopentad fraction [mmmm], and the racemic pentad fraction [rrrr] and the racemic meso racemic mesopentad fraction [rmrm] described later are the meso fraction, the racemic fraction and the racemic meso racemic meso fraction, respectively, of pentad units in polypropylene molecule chains measured with respect to methyl group signals in a $^{13}$C-NMR spectrum in accordance with the method proposed in A. Zambelli, et al., "Macromolecules, 6, 925 (1973)". The larger the mesopentad fraction [mmmm] is, the higher the stereoregularity is. Triad fractions [mm], [rr] and [mr], which will be described later, are also calculated by the above method.

A $^{13}$C-NMR spectrum can be measured using the following apparatus under the following conditions according to the assignment of peaks proposed in A. Zambelli, et al., "Macromolecules, 8, 687 (1975)".

Apparatus: JNM-EX 400 $^{13}$C-NMR apparatus, manufactured by JEOL Ltd.
Method: Complete proton decoupling method
Concentration: 220 mg/ml
Solvent: 90 : 10 (by volume) mixed solvent of 1,2,4-trichlorobenzene and deuterated benzene
Temperature: 130°C

Pulse width: 45°
Pulse interval: 4 seconds
Integration: 10000 times

$$[\text{Calculation Formulae}]$$

$$M = m/S \times 100$$

$$R = \gamma/S \times 100$$

$$S = P\beta\beta + P\alpha\beta + P\alpha\gamma$$

S: signal intensity of methyl carbon atoms in side chains of all propylene units
$P\beta\beta$: 19.8 ppm to 22.5ppm
$P\alpha\beta$: 18.0 ppm to 17.5ppm
$P\alpha\gamma$: 17.5 ppm to 17.1ppm
$\Gamma$: racemic pentad chains: 20.7 ppm to 20.3 ppm
m: mesopentad chains: 21.7 ppm to 22.5 ppm

(b)

$$[\text{rrrr}]/(1 - [\text{mmmm}]) \leq 0.1$$

The [rrrr]/[1 - mmmm] value is obtained from the above pentad fractions. This value is an index of the uniformity of regularity distribution in the component unit derived from propylene in the polymer (II). As this value increases, the polymer is a mixture of high-regularity polypropylene and atactic polypropylene as is the case for conventional polypropylene produced using the existing catalyst system, which causes stickiness.
In a case in which the polymer (II) has [rrrr]/(1 - [mmmm]) of 0.1 or less, the stickiness of the obtained spunbonded nonwoven fabric is suppressed. From such a viewpoint, [rrrr]/(1 - [mmmm]) is preferably 0.05 or less, and more preferably 0.04 or less. The lower limit of [rrrr]/(1 - [mmmm]) is 0.01.
(c)

$$[\text{rmrm}] > 2.5 \text{ mol}\%;$$

In a case in which the racemic meso racemic meso fraction [rmrm] of the polymer (II) exceeds 2.5 mol%, the randomness of the polymer (II) is increased and the elastic recoverability of the spunbonded nonwoven fabric is further improved. [rmrm] is preferably 2.6 mol% or more, and more preferably 2.7 mol% or more. The upper limit of the racemic meso racemic meso fraction [rmrm] of the polymer (II) is usually about 10 mol%.
(d)

$$[\text{mm}] \times [\text{rr}]/[\text{mr}]^2 \leq 2.0$$

$[\text{mm}] \times [\text{rr}]/[\text{mr}]^2$ represents an index of the randomness of the polymer (II). In a case in which this value is 2.0 or less, the elastic nonwoven fabric has sufficient elastic recoverability and suppressed stickiness. As $[\text{mm}] \times [\text{rr}]/[\text{mr}]^2$ becomes closer to 0.25, the randomness is increased. From the viewpoint of obtaining the above sufficient elastic recoverability, $[\text{mm}] \times [\text{rr}]/[\text{mr}]^2$ is preferably from more than 0.25 to 1.8 or less, and more preferably from 0.5 to 1.5.
(e)

$$\text{weight-average molecular weight (Mw)} = 10{,}000 \text{ to } 200{,}000$$

In a case in which the weight-average molecular weight of the polymer (II) which is a propylene homopolymer is 10,000 or more, the polymer (II) exhibits a moderate viscosity not too low, so as to suppress filament breakage during the production of the spunbonded nonwoven fabric obtained by the composition. In a case in which the weight-average molecular weight is 200,000 or less, the polymer (II) exhibits a moderate viscosity not too high, so as to achieve improved spinnability. This weight-average molecular weight is preferably from 10,000 to 150,000. The measurement method of the weight-average molecular weight of the polymer (II) will be described later.

(f)

$$\text{molecular weight distribution } (Mw/Mn) < 4,$$

In a case in which the molecular weight distribution (Mw/Mn) of the polymer (II) is less than 4, the occurrence of the stickiness of the resulted spunbonded nonwoven fabric is suppressed. The molecular weight distribution is preferably from 1.5 to 3.

The weight-average molecular weight (Mw) is a weight-average molecular weight in terms of polystyrene measured by the gel permeation chromatography (GPC) method under the following apparatus and conditions. The molecular weight distribution (Mw/Mn) is a value calculated from the number average molecular weight (Mn) measured in the similar manner with the weight-average molecular weight (Mw), and the weight-average molecular weight (Mw).

[GPC Measuring Apparatus]

Column: TOSOGMHHR-H(S)HT
Detector: RI detector for liquid chromatogram WATERS 150C
[Measurement Conditions]
Solvent: 1,2,4-trichlorobenzene
Measurement temperature: 145°C
Flow rate: 1.0 ml/min
Sample concentration: 2.2 mg/ml
Injection volume: 160 μl
Calibration curve: Universal Calibration
Analysis program: HT-GPC (Ver. 1.0)

It is preferable that the polymer (II) further satisfies the following (g).
(g) melting point (Tm-D) = 0°C to 120°C;

The melting point (Tm-D) of the polymer (II) is a melting point (Tm-D) defined as the peak top temperature of the peak observed on the highest temperature side of the melting endothermic curve. The melting endothermic curve is obtained by using a differential scanning calorimeter (DSC), holding the polymer (II) at -10°C for 5 minutes under a nitrogen atmosphere and thereafter heating at a temperature increasing rate of 10°C/min.

In a case in which the melting point (Tm-D) of the polymer (II) is 0°C or higher, the occurrence of the stickiness of the spunbonded nonwoven fabric formed by the composition is suppressed. In a case in which the melting point is 120°C or less, sufficient elastic recoverability is obtained. From this viewpoint, the melting point (Tm-D) is more preferably from 0°C to 100°C, and further preferably from 30°C to 100°C.

The melting point (Tm-D) can be determined by using a differential scanning calorimeter (DSC-7, manufactured by Perkin Elmer), holding 10 mg of a sample at -10°C under a nitrogen atmosphere for 5 minutes, thereafter heating the sample at a temperature increase rate of 10°C/min to record a melting endothermic curve, and determining the temperature as the peak top of the peak observed on the highest temperature side of the curve.

From the viewpoint of obtaining the favorable spinnability and the excellent drawing processability, in general, the melt flow rate (MFR, under measurement conditions of a temperature of 230°C and a load of 2160 g) of the polymer (II), which is measured by a method in accordance with ASTM D 1238, is preferably in a range of from 1 g/10 min to 100 g/10 min, more preferably in a range of from 5 g/10 min to 100 g/10 min, and still more preferably in a range of from 30 g/10 min to 70 g/10 min.

The polymer (II) can be synthesized using a homogeneous catalyst referred to as a so-called metallocene catalyst as described, for example, in International Publication No. 2003/087172.

[Additive]

The composition may contain various known additives such as an antioxidant, a heat stabilizer, a weathering stabilizer, an antistatic agent, a slip agent, an antifogging agent, a lubricant, a dye, a pigment, a natural oil, a synthetic oil, wax, and fatty acid amide as optional components while still achieving the object of the invention.

(Fatty Acid Amide)

As described above, it is preferable to contain a fatty acid amide having from 15 to 22 carbon atoms in a composition.

When the composition contains a fatty acid amide, the fatty acid amide is adsorbed onto a fiber surface of a spunbonded nonwoven fabric formed of the composition, the fiber surface is modified, and the flexibility, the tactile feeling, the blocking resistance and the like are further improved. As a result, it is considered that adhesion of nonwoven fabric fibers to members such as various kinds of rotating machines in a device to be used in emboss processing or the like is more effectively suppressed.

Examples of the fatty acid amide having from 15 to 22 carbon atoms include a fatty acid monoamide compound, a fatty acid diamide compound, a saturated fatty acid monoamide compound, and an unsaturated fatty acid diamide compound.

In this regard, the number of carbon atoms of the fatty acid amide in the present specification means the total number of carbon atoms contained in the molecule, and the carbon atoms in -CONH constituting the amide is also included in the number of carbon atoms. The number of carbon atoms of the fatty acid amide is more preferably from 18 to 22.

Specific examples of the fatty acid amide include a palmitic acid amide (the number of carbon atoms: 16), a stearic acid amide (the number of carbon atoms: 18), an oleic acid amide (the number of carbon atoms: 18), and an erucic acid amide (the number of carbon atoms: 22).

Only one kind of fatty acid amide may be used, or two or more kinds of fatty acid amides may also be used, in a composition.

A preferable range of the content of the fatty acid amide with respect to the total amount of the composition is as described above.

<Method For Producing Spunbonded Nonwoven Fabric>

The spunbonded nonwoven fabric of the embodiment can be produced by a routine procedure using one kind or two or more kinds of the compositions described above.

Specifically, it is preferable that the spunbonded nonwoven fabric of the embodiment is produced by a method for producing a spunbonded nonwoven fabric of the embodiment shown below.

The method for producing a spunbonded nonwoven fabric of the embodiment is a method for producing a spunbonded nonwoven fabric, including a filtration step of melting a polyethylene at from 180°C to 200°C, and passing the melted polyethylene through a sieve that has an opening of 65 µm or less and has been heated to from 180°C to 200°C; a mixing step of obtaining a composition by mixing the polyethylene passed through the sieve in the filtration step, a propylene homopolymer having a melting point of 140°C or higher, and a specific polymer; and a nonwoven fabric forming step of obtaining a nonwoven fabric by a spunbond method, from the composition obtained in the mixing step, in which the number of the particles each having a diameter of 0.2 mm or less contained in a film formed of the polyethylene that has passed through the sieve in the filtration step is 25 pieces/g or less.

Hereinafter, each of the steps will be described.

[Filtration Step]

In a filtration step, a polyethylene is melted at from 180°C to 200°C, and the melted polyethylene is passed through a sieve having an opening of 65 µm or less.

When the melting temperature of the polyethylene is 180°C or higher, clogging of the sieve hardly occurs, and the filtration step is stably performed. Further, when the melting temperature of the polyethylene is 200°C or less, deterioration of the polyethylene is suppressed, and a spunbonded nonwoven fabric having excellent extensibility can be obtained. For the similar reason, the melting temperature of the polyethylene is preferably from 185°C to 195°C.

In this step, a sieve with an opening of 65 µm or less is used. From the viewpoint of obtaining a spunbonded nonwoven fabric having more excellent extensibility, the sieve has an opening of preferably 62 µm or less, and more preferably 59 µm or less.

In addition, from the viewpoint of the efficiency in the filtration step, the sieve has an opening of preferably 30 µm or more, and more preferably 40 µm or more.

By filtering the melted polyethylene through a sieve with an opening of 65 µm or less heated to the above temperature, the length of an island phase in a fiber as a component of the spunbonded nonwoven fabric is shortened, and the average length of the island phase of 100 µm or less is achieved.

The sieve used in this step is preferably heated before being brought into contact with the melted polyethylene.

Specifically, the sieve is more preferably heated to from 100°C to 300°C, and more preferably heated to from 150°C to 220°C.

By heating the sieve, clogging of the sieve tends to be suppressed.

The sieve used in this step is not limited as long as the sieve has an opening of 65 μm or less, and the sieve is preferably made of metal (for example, made of stainless steel) so as to withstand the heating.

The polyethylene passed through the sieve in this step satisfies the following physical properties.

That is, the number of the particles each having a diameter of 0.2 mm or less contained in a film formed of the polyethylene passed through the sieve is 25 pieces/g or less, preferably 20 pieces/g or less, and more preferably 15 pieces/g or less.

Hereinafter, the "particles having a diameter of 0.2 mm or less" contained in a film formed of the polyethylene are referred to as "small particles".

When the number of the small particles is 25 pieces/g or less, the length of an island phase in a fiber as a component of the obtained spunbonded nonwoven fabric is shortened, and improvement in the extensibility can be achieved.

Further, the number of the particles having a diameter exceeding 2 mm contained in a film formed of the polyethylene passed through the sieve is preferably 10 pieces/g or less, more preferably 5 pieces/g or less, and still more preferably 1 piece/g or less.

Hereinafter, the "particles having a diameter exceeding 2 mm" contained in a film formed of the polyethylene are referred to as "large particles".

When the number of the large particles is 10 pieces/g or less, the thread breakage during spinning is easily suppressed, the spinnability is excellent, and improvement in productivity can be achieved.

The number of the particles contained in a film formed of the above polyethylene is determined as follows.

That is, the polyethylene passed through the sieve in this step is melt-kneaded at 280°C for 30 minutes with an extruder, and the molten polyethylene is extruded through a **T**-die at 280°C to obtain a film having a thickness of 55 μm.

The obtained film was cut into pieces of 100 cm × 25 cm, and the pieces are weighed, and used as measurement samples. As the measurement samples, three pieces are collected.

For each of the obtained three pieces of measurement samples, the surface is visually observed, and the diameter of a particle found is observed and confirmed with an optical microscope. The diameter of the particles contained in each of the measurement samples is measured, each of the particles is classified as either a large particle or a small particle, and the number of each of the large particles and the small particles is counted. By dividing the total number of the large particles or the small particles obtained from the three pieces of measurement samples by the total weight of the three pieces of measurement samples, the number of the large particles or the small particles, per 1 g of the measurement samples is determined.

[Mixing Step]

In a mixing step, by mixing a polyethylene passed through a sieve in a filtration step, a propylene homopolymer having a melting point of 140°C or higher, and a specific polymer, a composition is obtained.

Further, components other than the polyethylene, used in this step, that is, the propylene homopolymer having a melting point of 140°C or higher, the specific polymer, and an additive agent (optional component such as a fatty acid amide having from 15 to 22 carbon atoms) each may be in a molten state or in a solid state.

This step may be performed in an extruder used in a nonwoven fabric forming step.

The content of the polyethylene used in this step is from 1.0% by mass to 15.0% by mass with respect to the total amount of the composition.

Further, the content of the specific polymer used in this step is from 5% by mass to 30% by mass with respect to the total amount of the composition.

In addition, the content of the propylene homopolymer having a melting point of 140°C or higher used in this step is from 55.0% by mass to 90.0% by mass with respect to the total amount of the composition.

Moreover, this step is preferably a step of obtaining a composition by further mixing a fatty acid amide having from 15 to 22 carbon atoms.

That is, this step is preferably a step of obtaining a composition by mixing a polyethylene passed through a sieve in a filtration step, a propylene homopolymer having a melting point of 140°C or higher, a specific polymer, and a fatty acid amide having from 15 to 22 carbon atoms.

In addition, the content of the fatty acid amide having from 15 to 22 carbon atoms used in this step is preferably from 0.1% by mass to 5.0% by mass with respect to the total amount of the composition.

[Nonwoven Fabric Forming Step]

In a nonwoven fabric forming step, a nonwoven fabric is obtained, by a spunbond method, from the composition obtained in the mixing step.

In this step, a nonwoven fabric is obtained by, for example, the following method.

That is, a method in which a composition obtained in a mixing step is melted by using an extruder, the molten composition is melt spun by using a spunbonded nonwoven fabric forming machine having plural spinnerets, long fibers formed by the spinning are cooled if necessary, and then the long fibers are deposited on a collection surface of the spunbonded nonwoven fabric forming machine, and are subjected to heat and pressure treatment with embossing rolls is used.

The melting temperature of a composition is not particularly limited as long as the melting temperature is a softening or melting temperature or higher but less than a thermal decomposition temperature of a composition used for spinning, and the melting temperature may be appropriately determined depending on the physical properties and the like of a composition to be used.

Although depending on a composition to be used, the temperature of a spinneret is preferably from 180°C to 240°C, more preferably from 190°C to 230°C, and still more preferably from 200°C to 225°C, since the composition to be used in this step is a composition having a high content of a propylene-containing polymer.

In a case in which spun long fibers are cooled, it is preferred to use a technique of blowing cooling air to the long fibers so that the long fibers are drawn while being cooled.

The temperature of the cooling air for cooling the spun long fibers is not particularly limited as long as the temperature is a temperature at which the composition is solidified. In general, the temperature of the cooling air is in a range of preferably from 5°C to 50°C, more preferably from 10°C to 40°C, and still more preferably from 15°C to 30°C.

In a case in which spun long fibers are drawn by cooling air, in general, the wind speed of the cooling air is in a range of from 100 m/min to 10,000 m/min, and preferably in a range of from 500 m/min to 10,000 m/min.

Hereinafter, a nonwoven fabric forming step will be described in detail with reference to drawings.

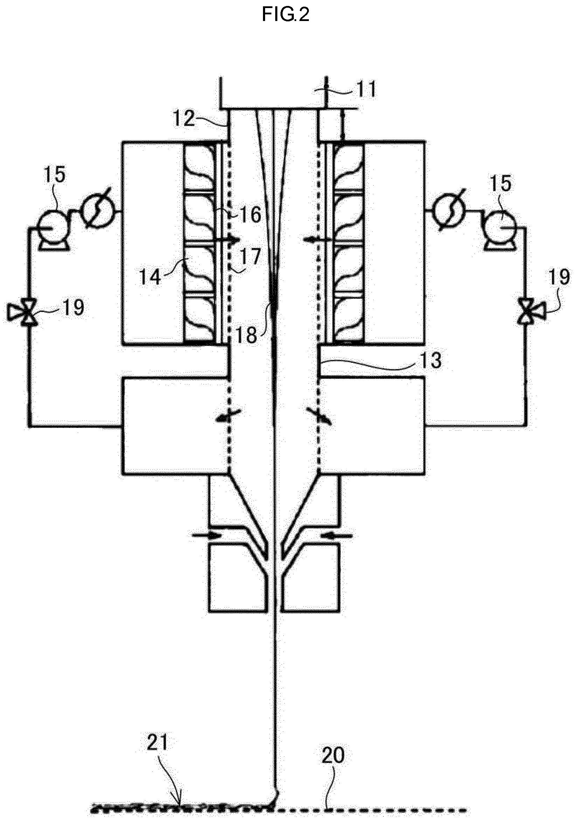

Fig. 2 is a schematic diagram of a closed-system spunbond method in which by using a composition that is a raw material of a nonwoven fabric, melt-spun long fibers are drawn while being cooled in an enclosed space, and as a result of which a spunbonded nonwoven fabric is produced.

The spunbond method shown in Fig. 2 is performed by a closed-system spunbonded nonwoven fabric production device having an enclosed space (closed cooling chamber 13). Specifically, in a closed-system spunbond method shown in Fig. 2, at first, a composition discharged through a spinneret 11 passes through a neck part 12 and is cooled in a closed cooling chamber 13, in a closed-system spunbonded nonwoven fabric production device, and thus long fibers 18 are formed. The formed long fibers 18 reach a collection device 20 and are deposited on a surface of the collection device 20, and as a result of which a spunbonded nonwoven fabric 21 is formed.

In this regard, into the cooling chamber 13, cooling air is supplied from a blower 15 equipped with a looper 14 through a filter 17. The supply amount of the cooling air into the cooling chamber 13 is adjusted by a blower 15, a switching valve 19 for adjusting the cooling air to be sent to the blower 15, and opening and closing of a damper 16.

This step has been described by taking a closed-system spunbond method as an example, however, this step is not limited to the closed-system spunbond method, and may also be, for example, an open-system spunbond method in which cooling is performed in an open space.

Fibers of a spunbonded nonwoven fabric to be obtained in this step may be partly thermally fused. Further, fibers of the spunbonded nonwoven fabric to be obtained in this step may be compacted by using a nip roll before being thermally fused.

As described above, a spunbonded nonwoven fabric of the embodiment can be obtained by passing through a filtration step, a mixing step, and a nonwoven fabric forming step.

[Nonwoven Fabric Multilayer]

A spunbonded nonwoven fabric of the embodiment may be used alone, or may be a nonwoven fabric multilayer in which a spunbonded nonwoven fabric of the embodiment and another layer are layered one on another, depending on the intended purpose.

In a case in which a nonwoven fabric multilayer is obtained by using a spunbonded nonwoven fabric of the embodiment, the another layer other than the spunbonded nonwoven fabric of the embodiment may contain one layer or two or more layers.

Specific examples of the another layer other than the spunbonded nonwoven fabric of the embodiment include a knit fabric, a woven fabric, a nonwoven fabric other than the spunbonded nonwoven fabric of the embodiment, and a film.

The method for further layering (sticking) the another layer on the spunbonded nonwoven fabric of the embodiment is not particularly limited, and various methods including a thermal fusion method such as thermal embossing, or ultrasonic fusion bonding, a mechanical entangling method such as needle punching, or water jetting, a method for using an adhesive agent such as a hot-melt adhesive, or a urethane-based adhesive, and extrusion lamination can be employed.

Examples of other nonwoven fabrics which can form a nonwoven fabric multilayer by layered with the spunbonded nonwoven according to the embodiments include various known nonwoven fabrics such as a spunbonded nonwoven fabric other than the spunbonded nonwoven fabric according to the embodiments, a meltblown nonwoven fabric, a wet nonwoven fabric, a dry nonwoven fabric, a dry pulp nonwoven fabric, a flash spun nonwoven fabric, and a split non-woven fabric. The nonwoven fabric may be a stretchable nonwoven fabric or a nonstretchable nonwoven fabric. Herein, the nonstretchable nonwoven fabric refers to a nonwoven fabric which does not cause stress at recovering after extending in MD (machine direction of the nonwoven fabric, longitudinal direction) or CD (direction perpendicular to the machine direction of the nonwoven fabric, lateral direction).

As a film that can be layered with a spunbonded nonwoven fabric of the embodiment to form a nonwoven fabric multilayer, in a case in which the nonwoven fabric multilayer requires air breathability, an air breathable film, or a moisture permeable film is preferable.

Examples of the air breathable film include various known air breathable films such as a film made of moisture permeable thermoplastic elastomer such as a polyurethane elastomer, a polyester elastomer, or a polyamide elastomer, and a porous film obtained by drawing a film made of a thermoplastic resin containing inorganic fine particles or organic fine particles to create pores in the film. As the thermoplastic resin used for the porous film, polyolefins such as high pressure low density polyethylene, linear low density polyethylene (so-called LLDPE), high density polyethylene, polypropylene, a polypropylene random copolymer, and combinations thereof are preferable.

Further, in a case in which the nonwoven fabric multilayer does not require air breathability, a film made of one or more kinds of thermoplastic resins selected from a polyethylene, a polypropylene, and the like can be used.

As a thermal fusion method in a case in which a nonwoven fabric multilayer is partly thermally fused, various known methods, for example, a method for using a means such as ultrasonic waves or the like, thermal embossing using embossing rolls, and hot air through can be mentioned.

Among them, thermal embossing is preferable because the long fibers are efficiently drawn when the nonwoven fabric multilayer is drawn.

In a case in which a part of the nonwoven fabric multilayer is thermally fusion bonded by thermal embossing, the emboss area ratio is usually from 5% to 30%, and preferably from 5% to 20%, and the non-embossed unit area is 0.5 $mm^2$ or more, and preferably from 4 $mm^2$ to 40 $mm^2$. The non-embossed unit area is the maximum area of a quadrangle inscribed in emboss in a non-embossed portion of minimum units surrounded by embossed portions in all directions. Examples of the shape of the mark include a circle, an ellipse, an oval, a square, a rhombus, a rectangle, a square, and continuous shapes based on these shapes.

By drawing the obtained nonwoven fabric multilayer, a stretchable nonwoven fabric multilayer having stretchability can be obtained.

The drawing method is not particularly limited, and conventionally known methods can be applied. The drawing method may be such that the nonwoven fabric multilayer is drawn partially or entirely. The drawing method may be such that the nonwoven fabric multilayer is drawn uniaxially or biaxially. Examples of the method of drawing the nonwoven fabric multilayer in a machine direction (MD) include a method of passing the partially fusion bonded mixed fibers through two or more pairs of nip rolls. At this time, the partially fusion bonded nonwoven fabric multilayer can be drawn by increasing the rotational speeds of the nip rolls in order in the machine direction. Gear drawing can also be performed using a gear drawing device.

The draw ratio is preferably 50% or more, more preferably 100% or more, and further preferably 200% or more, and preferably 1000% or less, and more preferably 400% or less.

In the case of uniaxial drawing, the above draw ratio is preferably satisfied in the machine direction (MD) or a direction perpendicular thereto (CD). In the case of biaxial drawing, the above draw ratio is preferably satisfied in at least one of the machine direction (MD) or the direction perpendicular thereto (CD).

By drawing at the above draw ratio, the long fibers having the elastisity in the spunbonded nonwoven fabric are drawn. The long fibers which do not have stretchability are plastically deformed to be extended in accordance with the above draw ratio.

Further, similarly, also in the other layers to be layered, a layer having elasticity is elastically deformed, and a layer having no elasticity is plastically deformed.

During the forming of a nonwoven fabric multilayer, a layer having elasticity and a layer having no elasticity are layered one on another, the layered material is drawn, and after that when the stress is released, the layer having elasticity (long fibers as a component of the layer) is elastically recovered, and long fibers having no elasticity are not elastically recovered but are bent into folds, as a result of which a bulky feeling can be developed in the nonwoven fabric multilayer.

In addition, since the plastically deformed long fibers become thinner, the long fibers can have improved flexibility and tactile feeling, and an extension stop function.

<Hygiene Material>

A hygiene material of the embodiment contains the above-described spunbonded nonwoven fabric of the embodiment.

The spunbonded nonwoven fabric according to the invention is excellent in the extensibility. Therefore, the spunbonded nonwoven fabric of the embodiment is suitably used for a hygiene material.

Examples of the hygiene material include an absorbent article such as a paper diaper, or a sanitary napkin, a hygiene material for medical use such as a bandage, medical gauze, or a towel, and a hygiene mask.

The hygiene material in which a spunbonded nonwoven fabric of the embodiment can be contained is not limited to the examples mentioned above, and can be suitably used for any of various hygiene material applications where the extensibility and flexibility are required.

The hygiene material may contain a spunbonded nonwoven fabric of the embodiment as a nonwoven fabric multilayer containing the spunbonded nonwoven fabric of the embodiment and other layers.

EXAMPLES

Hereinafter, an embodiment according to the invention will be more specifically described with reference to Examples, but the invention is not limited to these Examples which are exemplary embodiments. Physical property values and the like in Examples and Comparative Examples were measured by the following methods.

(1) Average Length of Island Phase [$\mu$m]

A fiber was taken out from a spunbonded nonwoven fabric, the fiber was embedded in paraffin to prepare a measurement sample. Further, the measurement sample was placed on a microtome such that the blade was parallel to the major axis direction of the fiber, and the measurement sample was sliced along the major axis direction of the fiber. After that, the fiber obtained by slicing the measurement sample was carbon-reinforced, and then with regard to the cross section, the cross section of the fiber obtained by slicing the measurement sample was observed by using a transmission electron microscope (TEM), the length of each of arbitrary 100 island phases was measured, and the average length was determined.

In this regard, as the transmission electron microscope, transmission electron microscope model: H-7650 manufactured by Hitachi High-Technologies Corporation was used, and the observation magnification was 8000-fold magnification.

(2) Basis Weight [g/m$^2$]

Ten test pieces of 300 mm (machine direction: MD) $\times$ 250 mm (lateral direction: CD) were sampled from a spunbonded nonwoven fabric. Ten sampling points were arbitrarily selected. Subsequently, the mass (g) of each of the sampled test pieces was measured using a scale electric balance (manufactured by Kensei Co., Ltd.). The average value of the masses of the test pieces was determined. The determined average value was converted to the mass (g) per 1 m$^2$ by rounding off to unit to give a basis weight [g/m$^2$] of each spunbonded nonwoven fabric.

(3) Maximum Elongation [%] and Maximum Strength [N/50 mm]

From a spunbonded nonwoven fabric, the maximum elongation and the maximum strength were measured as follows in accordance with 6.12.1 [method A] of JIS L 1906.

In a thermostatic chamber at a temperature of 20 $\pm$ 2°C and a humidity of 65 $\pm$ 2% described in JIS Z 8703 (Standard Atmospheric Conditions for Testing), five test pieces each having 25 cm in a machine direction (so-called MD direction) $\times$ 5 cm in a lateral direction (so-called CD direction) were sampled. Under the conditions of a distance between chucks of 100 mm and a tensile rate of 300 mm/min, the obtained test pieces were subjected to a tensile test by using a tensile testing machine (INSTRON 5564, manufactured by Instron Japan Company, Ltd.), and a tensile load was measured for five test pieces. The average value of the maximum measured values was defined as the maximum strength [N/50 mm].

Further, the elongation at the maximum strength was defined as the maximum elongation [%].

(4) Evaluation of Heat Sealability

[Heat Sealing Method]

Ten test pieces each having 100 mm in a machine direction (so-called MD direction) $\times$ 100 mm in a lateral direction (so-called CD direction) were sampled from a spunbonded nonwoven fabric. Next, two of the test pieces were superposed on each other so that the MD directions of the two test pieces were the same directions, and were heat sealed by using a heat-seal testing machine (product name: Heat Seal Tester) manufactured by TESTER SANGYO CO., LTD. under the following conditions.

Heat-seal bar width: 10.0 mm
Heat-seal pressure: 2.0 kg/cm$^2$
Heat-seal time: 1.0 second
Heat-seal temperature: upper bar and lower bar were set at the same temperature (145°C or 155°C)
Heat-seal direction: perpendicular to the MD direction

[Confirmation of Heat Seal Strength]

By using a constant speed tensile testing machine (product name: STROGRAPH, manufactured by Toyo Seiki Seisaku-sho, Ltd.), a tensile peel test for each of five test pieces to which heat sealing had been performed under the above conditions was performed under the following conditions, the presence or absence of peeling was confirmed, and in a case in which there was no peeling, the test piece was evaluated as "having heat sealability".

Test piece shape: width of 20 mm, and length of 50 mm
Tensile rate: 30 mm/min
Atmospheric temperature during measurement: 23°C

(5) Emboss Remaining Rate [%]

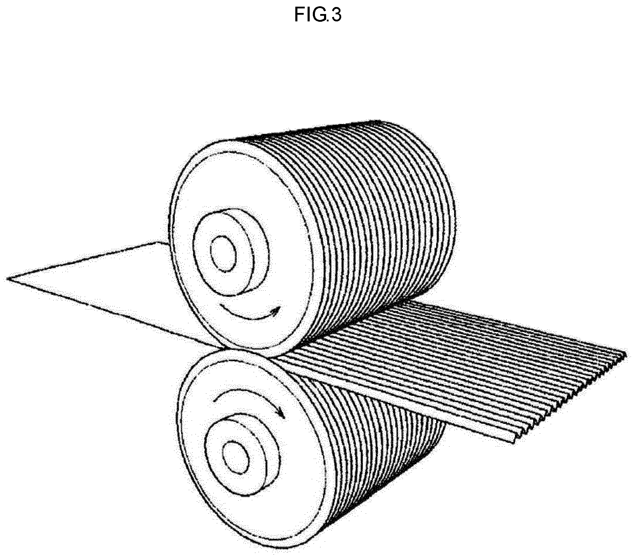

One test piece of 250 mm in a machine direction (so-called MD direction) $\times$ 200 mm in a lateral direction (so-called CD direction) was sampled from a spunbonded nonwoven fabric. The obtained test piece was inserted into a gear drawing device (that is, gear processing machine) shown in Fig. 3 such that a roll rotation direction of the gear drawing device and a CD direction of the test piece were matched, and a spunbonded nonwoven fabric gear-drawn in a MD direction (that is, direction of flow of the nonwoven fabric) was obtained. Each of the gear rolls mounted on a gear processing machine had a diameter of 200 mm and a gear pitch of 2.5 mm, and the engagement depth between both rolls was adjusted so as to be 5.5 mm.

The spunbonded nonwoven fabric that had been gear drawn as described above was subjected to morphological observation with a scanning electron microscope (SEM), and the emboss remaining rate after the gear drawing was evaluated. As the emboss remaining rate was higher, it was evaluated that the tactile feeling was more favorable. The emboss remaining rate was calculated by using the following Formula.

Emboss remaining rate = (the number of embossments not destroyed / the number of --> embossments observed) $\times$ 100

In addition, by observing an embossed part of the gear-drawn spunbonded nonwoven fabric with a SEM, an embossed part where none of the generation of holes in the embossed part, the detachment of fibers, and the fiber breakage in the embossed part and the boundary thereof were observed was defined as "undestroyed embossment".

The remaining rate of the embossments formed by the drawing processing using a gear processing machine was favorable, and it can be confirmed that since the fiber breakage of a spunbonded nonwoven fabric in an embossed part and the boundary thereof during drawing processing, and the breakage of the nonwoven fabric, caused by the fiber breakage were not generated, the drawing processability of a spunbonded nonwoven fabric was favorable.

(6) Flexibility Evaluation

For a spunbonded nonwoven fabric, sensory evaluation of tactile feeling (touch) was performed by touching the spunbonded nonwoven fabric directly by hand, and evaluation was made on the basis of the following criteria. The sensory evaluation was performed by 10 monitors, and the result that had been obtained from the largest number of monitors was adopted as the evaluation result.

In this regard, in a case in which there were two or more of the results that had been obtained from the largest number of monitors, the better result was adopted as the evaluation result.

-Evaluation criteria-

A: Touch was extremely favorable and flexibility was excellent.
B: Touch was favorable and flexibility was more excellent as compared with that in the following C evaluation.
C: Touch was stiff and flexibility was poor.

[Example 1]

<Production of Spunbonded Nonwoven Fabric>

-Filtration Step-

At first, a high density polyethylene having a MFR (measured at a temperature of 190°C under a load of 2.16 kg in accordance with ASTM D 1238) of 5 g/10 min, a density of 0.95 g/cm$^3$, and a melting point of 134°C was heated to 190°C and melted.
Subsequently, the high density polyethylene was filtered through a metal sieve (with an opening of 58 $\mu$m) heated to 190°C while remaining in a molten state.
The number of each of the small particles and the large particles, which were contained in a film formed of the polyethylene passed through the sieve in the above filtration step was determined by the method described above.
The results are shown in Table 1.

-Mixing Step-

In the above filtration step, 7% by mass of polyethylene passed through a sieve, 72.7% by mass of propylene homopolymer having a MFR (measured at a temperature of 230°C under a load of 2.16 kg in accordance with ASTM D 1238) of 60 g/10 min, a density of 0.91 g/cm$^3$, and a melting point of 160°C, 20% by mass of propylene random copolymer (copolymer of propylene and ethylene having a polymerization molar ratio of 97 : 3, polymer (I)) having a MFR (measured at a temperature of 230°C under a load of 2.16 kg in accordance with ASTM D 1238) of 60 g/10 min, a density of 0.91 g/cm$^3$, and a melting point of 142°C, and 0.3% by mass of erucic acid amide were mixed, and a composition was obtained.

-Nonwoven Fabric Forming Step-

The composition obtained in the above mixing step was melted by using an extruder with 75 mm$\phi$, and by using a spunbonded nonwoven fabric production device having a spinneret with 2557 holes (device used in the closed-system spunbond method shown in Fig. 2, having a length of 800 mm in a direction perpendicular to the machine flow direction on a collection surface), melt spinning was performed by a closed-system spunbond method under the conditions that the melting temperature of a composition and the die temperature were both 220°C, the cooling air temperature was 20°C, and the drawing air wind speed was 5233 m/min.
The spun long fibers were deposited on a collection surface, and by performing heat and pressure treatment (with an embossed area ratio (thermocompression bonding ratio) of 18% and an embossing temperature of 116°C) with embossing rolls on the long fibers, a spunbonded nonwoven fabric having a total weight per unit area of 18 g/m$^2$ was prepared.
The obtained spunbonded nonwoven fabric of Example 1 was evaluated by the evaluation method described above.
The results are shown in the following Table 1.

[Example 2]

<Production of Spunbonded Nonwoven Fabric>

A polyethylene was filtered in a similar manner as in Example 1 except that a metal sieve with an opening of 64 $\mu$m was used in place of the metal sieve with an opening of 58 $\mu$m in the filtration step in the production of the spunbonded nonwoven fabric of Example 1, and then, by using the filtered polyethylene, a composition was obtained, and subsequently, a spunbonded nonwoven fabric was prepared.
The obtained spunbonded nonwoven fabric of Example 2 was evaluated by the evaluation method described above.
The results are shown in the following Table 1.

<Preparation of Low Crystalline Polypropylene >

To a stainless steel reactor having an inner volume of 0.2 m$^3$ equipped with a stirrer, n-heptane at 20 L/h, triisobutylaluminum at 15 mmol/h, and a catalyst component obtained by preliminarily contacting dimethylanilinium tetrakispentafluorophenylborate, (1,2'-dimethylsilylene)(2,1'-dimethylsilylene)-bis(3-trimethylsilylmethylindenyl)zirconium dichloride, triisobutylaluminum, and propylene at 6 μmol/h per zirconium were continuously supplied.

Propylene and hydrogen were continuously supplied in a state where a hydrogen concentration in a gas phase portion was 8 mol% at a polymerization temperature of 70°C and the total pressure in the reactor was maintained at 0.7 MPa·G.

To the obtained polymerization solution, 1000 ppm of SUMILIZER GP (manufactured by Sumitomo Chemical Co., Ltd.) was added, and the solvent was removed to obtain a low crystalline propylene polymer.

The low crystalline propylene polymer thus obtained had a weight-average molecular weight (Mw) of $1.2 \times 10^4$ and Mw/Mn of 2. According to NMR measurement, [mmmm] was 46 mol%; [rrrr]/(1 - [mmmm]) was 0.038; [rmrm] was 2.7 mol%; and [mm] $\times$ [rr]/[mr]$^2$ was 1.5.

The low crystalline polypropylene (polymer (II)) thus obtained is described hereinafter as "LMPP1".

<Production of Spunbonded Nonwoven Fabric>

A composition was obtained in a similar manner as in Example 1 except that 20% by mass of the LMPP1 synthesized in the above was used in place of the 20% by mass of propylene random copolymer (copolymer of propylene and ethylene having a polymerization molar ratio of 97 : 3, polymer (I)) having a MFR (measured at a temperature of 230°C under a load of 2.16 kg in accordance with ASTM D 1238) of 60 g/10 min, a density of 0.91 g/cm$^3$, and a melting point of 142°C in the mixing step in the production of the spunbonded nonwoven fabric of Example 1, and subsequently, a spunbonded nonwoven fabric was prepared.

The obtained spunbonded nonwoven fabric of Example 3 was evaluated by the evaluation method described above.

The results are shown in the following Table 1.

[Example 4]

<Production of Spunbonded Nonwoven Fabric>

A composition was obtained in a similar manner as in Example 1 except that the amount of a propylene homopolymer having a MFR (measured at a temperature of 230°C under a load of 2.16 kg in accordance with ASTM D 1238) of 60 g/10 min, a density of 0.91 g/cm$^3$, and a melting point of 160°C was changed to 74.7% by mass, and the amount of the filtered polyethylene was changed to 5% by mass in the mixing step in the production of the spunbonded nonwoven fabric of Example 1, and subsequently, a spunbonded nonwoven fabric was prepared.

The obtained spunbonded nonwoven fabric of Example 4 was evaluated by the evaluation method described above.

The results are shown in the following Table 1.

[Comparative Example 1]

<Production of Spunbonded Nonwoven Fabric>

A polyethylene was filtered in a similar manner as in Example 1 except that a metal sieve with an opening of 69 μm was used in place of the metal sieve with an opening of 58 μm in the filtration step in the production of the spunbonded nonwoven fabric of Example 1, and then, by using the filtered polyethylene, a composition was obtained, and subsequently, a spunbonded nonwoven fabric was prepared.

The obtained spunbonded nonwoven fabric of Comparative Example 1 was evaluated by the evaluation method described above.

The results are shown in the following Table 1.

[Comparative Example 2]

<Production of Spunbonded Nonwoven Fabric>

A polyethylene was filtered in a similar manner as in Example 3 except that a metal sieve with an opening of 69 μm

was used in place of the metal sieve with an opening of 58 $\mu$m in the filtration step in the production of the spunbonded nonwoven fabric of Example 3, and then, by using the filtered polyethylene, a composition was obtained, and subsequently, a spunbonded nonwoven fabric was prepared.

The obtained spunbonded nonwoven fabric of Comparative Example 2 was evaluated by the evaluation method described above.

The results are shown in the following Table 1.

[Comparative Example 3]

<Production of Spunbonded Nonwoven Fabric>

A polyethylene was filtered in a similar manner as in Example 4 except that a metal sieve with an opening of 69 $\mu$m was used in place of the metal sieve with an opening of 58 $\mu$m in the filtration step in the production of the spunbonded nonwoven fabric of Example 4, and then, by using the filtered polyethylene, a composition was obtained, and subsequently, a spunbonded nonwoven fabric was prepared.

The obtained spunbonded nonwoven fabric of Comparative Example 3 was evaluated by the evaluation method described above.

The results are shown in the following Table 1.

In the following Table 1, conditions in a filtration step, the number of each of the small particles and the large particles, which are contained in a film formed of a polyethylene passed through a sieve in the above filtration step, an amount of each of the components to be added in a mixing step, conditions in a nonwoven fabric forming step, and measurement and evaluation results are shown.

Note that the expression "-" in Table 1 means that the corresponding component was not contained.

[Table 1]

| | | | Example 1 | Example 2 | Example 3 | Example 4 | Comparative Example 1 | Comparative Example 2 | Comparative Example 3 |
|---|---|---|---|---|---|---|---|---|---|
| Filtration step | | Opening of sieve [$\mu$m] | 58 | 64 | 58 | 58 | 69 | 69 | 69 |
| | | Melting temperature and sieve temperature [°C] | 190 | 190 | 190 | 190 | 190 | 190 | 190 |
| | | Number of small particles [pieces/g] | 4.1 | 18.4 | 6.2 | 6.1 | 184 | 180 | 179 |
| | | Number of large particles [pieces/g] | 0.2 | 1.2 | 0.3 | 0.2 | 6.8 | 5.3 | 5.5 |
| Mixing step | Component [% by mass] | Propylene homopolymer having melting point of 140°C or higher | 72.7 | 72.7 | 72.7 | 74.7 | 72.7 | 72.7 | 74.7 |
| | | Propylene random copolymer (polymer (I)) | 20 | 20 | - | 20 | 20 | - | 20 |
| | | LMPP1 (polymer (II)) | - | - | 20 | - | - | 20 | - |
| | | Polyethylene | 7 | 7 | 7 | 5 | 7 | 7 | 5 |
| | | Erucic acid amide | 0.3 | 0.3 | 0.3 | 0.3 | 0.3 | 0.3 | 0.3 |
| Nonwoven fabric forming step | | Embossing temperature [°C] | 116 | 116 | 116 | 116 | 116 | 116 | 116 |
| | | Basis weight [g/m$^2$] | 18 | 18 | 18 | 18 | 18 | 18 | 18 |
| Measurement and evaluation results | | Average length of island phase [$\mu$m] | 9.2 | 17.5 | 14.7 | 18.4 | 500< | 500< | 500< |
| | Maximum elongation | MD direction [%] | 183 | 161 | 174 | 157 | 151 | 160 | 144 |
| | | CD direction [%] | 194 | 173 | 174 | 162 | 160 | 161 | 148 |
| | Maximum strength | MD direction [N/50 mm] | 37 | 37 | 33 | 43 | 37 | 33 | 43 |
| | | CD direction [N/50 mm] | 12 | 13 | 8 | 14 | 13 | 7 | 14 |
| | | Heat sealability | Present | Present | Present | Present | Present | Present | Present |
| | | Emboss remaining rate | 57% | 58% | 93% | 59% | 57% | 92% | 57% |
| | | Flexibility (sensory evaluation) | A | A | A | A | A | A | A |

From the results in Table 1, it can be understood that the spunbonded nonwoven fabric of the embodiment obtained in each of Examples from 1 to 4 has excellent extensibility.

Further, as shown in the above Table 1, it can be understood that all of the spunbonded nonwoven fabrics in Examples 1 to 4 are excellent in the extensibility, and further are excellent in the drawing processability because of the favorable heat sealability and the favorable emboss remaining rate. In addition, it can also be understood that the spunbonded nonwoven fabrics in Examples 1 to 4 are all excellent in the flexibility.

From these evaluation results, it can be understood that the spunbonded nonwoven fabric of the embodiment is suitable for use in a hygiene material that requires extensibility, flexibility, and processability.

**Claims**

1.  A spunbonded nonwoven fabric, comprising:

    a fiber formed of a composition containing:

    a propylene homopolymer having a melting point of 140°C or higher;
    a polyethylene; and
    at least one polymer selected from the group consisting of a polymer shown in (I), and a polymer shown in (II),

    wherein the fiber has a sea-island structure,
    an average length of an island phase, as determined by the method indicated in the passage of the specification titled "(1) Average Length of Island Phase [$\mu$m]", is from 1 $\mu$m to 100 $\mu$m in a cross section taken along a major axis direction of the fiber,
    a content of the polyethylene is from 1.0 % by mass to 15.0 % by mass with respect to a total amount of the composition,
    a content of the at least one polymer selected from the group consisting of the polymer shown in (I) and the polymer shown in (II) is from 5.0 % by mass to 30.0 % by mass with respect to a total amount of the composition, and
    a content of the propylene homopolymer having a melting point of 140°C or higher is from 55.0 % by mass to 90.0 % by mass with respect to a total amount of the composition:

    (I) a random copolymer of a propylene with at least one selected from ethylene or $\alpha$-olefin having from 4 to 20 carbon atoms;
    (II) a propylene homopolymer having a melting point of less than 120°C satisfying the following (a) to (f):

    (a) [mmmm] = 20 mol% to 60 mol%;
    (b)

$$[rrrr]/(1 - [mmmm]) \leq 0.1;$$

    (c)

$$[rmrm] > 2.5 \text{ mol}\%;$$

    (d)

$$[mm] \times [rr]/[(mr)]^2 \leq 2.0;$$

    (e) weight-average molecular weight (Mw) = 10,000 to 200,000; and
    (f)

$$molecular\ weight\ distribution\ (Mw/Mn) < 4,$$

    wherein, in (a) to (d):

[mmmm] is a mesopentad fraction;
[rrrr] is a racemic pentad fraction;
[rmrm] is a racemic meso racemic mesopentad fraction; and
[mm], [rr] and [mr] are respectively triad fractions thereof, [mmmm], [rrrr], [rmrm], [mm], [rr] and [mr] being determined in accordance with the method proposed in A. Zambelli, et al., "Macromolecules, 6, 925 (1973)", and the molecular weight distribution (Mw/Mn) being determined by gel permeation chromatography (GPC).

2. The spunbonded nonwoven fabric according to claim 1, wherein a density of the polyethylene is from 0.941 g/cm$^3$ to 0.970 g/cm$^3$.

3. The spunbonded nonwoven fabric according to claim 1 or 2, wherein the composition contains a fatty acid amid having 15 to 22 carbon atoms, and a content of the fatty acid amid having 15 to 22 carbon atoms is from 0.1 % by mass to 5.0 % by mass with respect to a total amount of the composition.

4. The spunbonded nonwoven fabric according to any one of claims 1 to 3, wherein the polymer shown in (I) is a random copolymer containing at least a constituent unit derived from propylene and a constituent unit derived from ethylene.

5. A hygiene material comprising the spunbonded nonwoven fabric according to any one of claims 1 to 4.

6. A method for producing a spunbonded nonwoven fabric according to claim 1, the method comprising:

a filtration step in which polyethylene is melted at from 180°C to 200°C, and the melted polyethylene is passed through a sieve having an opening of 65 $\mu$m or less;
a mixing step in which the polyethylene passed through the sieve in the filtration step, a propylene homopolymer having a melting point of 140°C or higher, and at least one polymer selected from the group consisting of a polymer shown in (I), and a polymer shown in (II) are mixed to obtain a composition; and
a nonwoven fabric forming step in which a nonwoven fabric is obtained, by a spunbond method, from the composition obtained in the mixing step,
wherein a number of particles each having a diameter of 0.2 mm or less contained in a film formed of the polyethylene that has passed through the sieve in the filtration step is 25 pieces/g or less,
a content of the polyethylene used in the mixing step is from 1.0 % by mass to 15.0 % by mass with respect to a total amount of the composition,
a content of the at least one polymer selected from the group consisting of the polymer shown in (I) and the polymer shown in (II) used in the mixing step is from 5.0 % by mass to 30.0 % by mass with respect to a total amount of the composition, and
a content of the propylene homopolymer having a melting point of 140°C or higher used in the mixing step is from 55.0 % by mass to 90.0 % by mass with respect to a total amount of the composition:

(I) a random copolymer of a propylene with at least one selected from ethylene or $\alpha$-olefin having from 4 to 20 carbon atoms;
(II) a propylene homopolymer having a melting point of less than 120°C satisfying the following (a) to (f):

(a) [mmmm] = 20 mol% to 60 mol%;
(b)

$$[rrrr]/(1 - [mmmm]) \leq 0.1;$$

(c)

$$[rmrm] > 2.5 \text{ mol\%};$$

(d)

$$[mm] \times [rr]/[(mr)]^2 \leq 2.0;$$

(e)

$$\text{weight-average molecular weight (Mw)} = 10{,}000 \text{ to } 200{,}000;$$

and
(f)

$$\text{molecular weight distribution (Mw/Mn)} < 4,$$

wherein, in (a) to (d):

[mmmm] is a mesopentad fraction;
[rrrr] is a racemic pentad fraction;
[rmrm] is a racemic meso racemic mesopentad fraction; and
[mm], [rr] and [mr] are respectively triad fractions thereof,

[mmmm], [rrrr], [rmrm], [mm], [rr] and [mr] being determined in accordance with the method proposed in A. Zambelli, et al., "Macromolecules, 6, 925 (1973)", and the molecular weight distribution (Mw/Mn) being determined by gel permeation chromatography (GPC).

7. The method for producing a spunbonded nonwoven fabric according to claim 6, wherein the mixing step is a step of obtaining a composition by further mixing a fatty acid amid having 15 to 22 carbon atoms, and a content of the fatty acid amid having 15 to 22 carbon atoms is from 0.1 % by mass to 5.0 % by mass with respect to a total amount of the composition.

**Patentansprüche**

1. Spinnvliesstoff, umfassend:
eine Faser, die aus einer Zusammensetzung gebildet ist, die Folgendes enthält:

ein Propylenhomopolymer mit einem Schmelzpunkt von 140 °C oder höher;
ein Polyethylen; und
mindestens ein Polymer, ausgewählt aus der Gruppe, bestehend aus einem in (I) gezeigten Polymer und einem in (II) gezeigten Polymer,
wobei die Faser eine Seeinselstruktur aufweist,
eine durchschnittliche Länge einer Inselphase, die durch das in dem Abschnitt der Beschreibung mit dem Titel "(1) Durchschnittliche Länge der Inselphase [$\mu$m]" angegebene Verfahren bestimmt wird, in einem Querschnitt entlang einer Hauptachsenrichtung der Faser 1 $\mu$m bis 100 $\mu$m beträgt,
ein Polyethylengehalt zwischen 1,0 Masse-% und 15,0 Masse-% der Gesamtmenge der Zusammensetzung beträgt,
ein Gehalt des mindestens einen Polymers, ausgewählt aus der Gruppe, bestehend aus dem in (I) gezeigten Polymer und dem in (II) gezeigten Polymer, von 5,0 Masse-% bis 30,0 Masse-%, bezogen auf eine Gesamtmenge der Zusammensetzung, beträgt, und
ein Gehalt des Propylenhomopolymers, das einen Schmelzpunkt von 140 °C oder höher aufweist, von 55,0 Masse-% bis 90,0 Masse-%, bezogen auf eine Gesamtmenge der Zusammensetzung beträgt:

(I) ein zufälliges Copolymer eines Propylens mit mindestens einem, ausgewählt aus Ethylen oder $\alpha$-Olefin, das 4 bis 20 Kohlenstoffatome aufweist;
(II) ein Propylenhomopolymer mit einem Schmelzpunkt von weniger als 120 °C, das die folgenden Punkte (a) bis (f) erfüllt:

(a) [mmmm] = 20 Mol-% bis 60 Mol-%;
(b)

$$[\text{rrrr}]/(1 - [\text{mmmm}]) \leq 0{,}1;$$

(c)

$$[rmrm] > 2{,}5 \text{ Mol\%;}$$

(d)

$$[mm] \times [rr]/[(mr)]^2 \leq 2{,}0;$$

(e)

$$\text{gewichtsmittleres Molekulargewicht (Mw)} = 10.000 \text{ bis } 200.000;$$

und
(f)

$$\text{Molekulargewichtsverteilung (Mw/Mn)} < 4,$$

wobei, in (a) bis (d):

[mmmm] eine Mesopentadenfraktion ist;
[rrrr] eine razemische Pentadenfraktion ist;
[rmrm] eine racemische meso racemische Mesopentadenfraktion ist; und
[mm], [rr] und [mr] jeweils Triadenfraktionen davon sind,

wobei [mmmm], [rrrr], [rmrm], [mm], [rr] und [mr] gemäß dem in A. Zambelli, et al., "Macromolecules, 6, 925 (1973)" vorgeschlagenen Verfahren bestimmt werden und die Molekulargewichtsverteilung (Mw/Mn) durch Gelpermeationschromatographie (GPC) bestimmt wird.

2. Spinnvliesstoff nach Anspruch 1, wobei die Dichte des Polyethylens zwischen 0,941 g/cm$^3$ und 0,970 g/cm$^3$ liegt.

3. Spinnvliesstoff nach Anspruch 1 oder 2, wobei die Zusammensetzung eine Fettsäureamid mit 15 bis 22 Kohlenstoffatomen aufweist und der Gehalt der Fettsäureamid mit 15 bis 22 Kohlenstoffatomen 0,1 bis 5,0 Masse-%, bezogen auf die Gesamtmenge der Zusammensetzung, beträgt.

4. Spinnvliesstoff nach einem der Ansprüche 1 bis 3, wobei das in (I) gezeigte Polymer ein statistisches Copolymer ist, das mindestens eine von Propylen abgeleitete Grundeinheit und eine von Ethylen abgeleitete Grundeinheit enthält.

5. Hygienematerial, umfassend den Spinnvliesstoff nach einem der Ansprüche 1 bis 4.

6. Verfahren zur Herstellung eines Spinnvliesstoffs nach Anspruch 1, das Verfahren umfassend:

einen Filtrationsschritt, bei dem Polyethylen bei 180 °C bis 200 °C geschmolzen wird und das geschmolzene Polyethylen durch ein Sieb mit einer Öffnung von 65 μm oder weniger geleitet wird;
einen Mischschritt, in dem das Polyethylen, das durch das Sieb im Filtrationsschritt hindurchgegangen ist, ein Propylenhomopolymer mit einem Schmelzpunkt von 140 °C oder höher und mindestens ein Polymer, ausgewählt aus der Gruppe, bestehend aus einem in (I) gezeigten Polymer und einem in (II) gezeigten Polymer, gemischt werden, um eine Zusammensetzung zu erhalten; und
einen Schritt zur Bildung eines Vliesstoffs, in dem ein Vliesstoff durch ein Spinnvliesverfahren aus der in dem Mischungsschritt erhaltenen Zusammensetzung erhalten wird,
wobei eine Anzahl von Partikeln, die jeweils einen Durchmesser von 0,2 mm oder weniger aufweisen und in einem aus dem Polyethylen gebildeten Film enthalten sind, der das Sieb im Filtrationsschritt passiert hat, 25 Stück/g oder weniger beträgt,
ein Gehalt des in dem Mischungsschritt verwendeten Polyethylens von 1,0 Massen-% bis 15,0 Massen-%, bezogen auf eine Gesamtmenge der Zusammensetzung, beträgt,
ein Gehalt des mindestens einen Polymers, ausgewählt aus der Gruppe, bestehend aus dem in (I) gezeigten Polymer und dem in (II) gezeigten Polymer, das in dem Mischungsschritt verwendet wird, von 5,0 Masse-% bis 30,0 Masse-%, bezogen auf eine Gesamtmenge der Zusammensetzung, beträgt, und
ein Gehalt des in dem Mischungsschritt verwendeten Propylenhomopolymers mit einem Schmelzpunkt von 140 °C oder höher von 55,0 Massen-% bis 90,0 Massen-%, bezogen auf eine Gesamtmenge der Zusammensetzung aufweist:

(I) ein zufälliges Copolymer eines Propylens mit mindestens einem, ausgewählt aus Ethylen oder $\alpha$-Olefin, das 4 bis 20 Kohlenstoffatome aufweist;
(II) ein Propylenhomopolymer mit einem Schmelzpunkt von weniger als 120 °C, das die folgenden Punkte (a) bis (f) erfüllt:

(a)

$$[mmmm] = 20 \text{ Mol-\%} \text{ bis } 60 \text{ Mol-\%};$$

(b)

$$[rrrr]/(1 - [mmmm]) \leq 0,1;$$

(c)

$$[rmrm] > 2,5 \text{ Mol\%};$$

(d)

$$[mm] \times [rr]/[(mr)]^2 \leq 2,0;$$

(e)

$$\text{gewichtsmittleres Molekulargewicht (Mw)} = 10.000 \text{ bis } 200.000;$$

und
(f)

$$\text{Molekulargewichtsverteilung (Mw/Mn)} < 4,$$

wobei, in (a) bis (d):

[mmmm] eine Mesopentadenfraktion ist;
[rrrr] eine razemische Pentadenfraktion ist;
[rmrm] eine racemische meso racemische Mesopentadenfraktion ist; und
[mm], [rr] und [mr] jeweils Triadenfraktionen davon sind,

wobei [mmmm], [rrrr], [rmrm], [mm], [rr] und [mr] gemäß dem in A. Zambelli, et al., "Macromolecules, 6, 925 (1973)" vorgeschlagenen Verfahren bestimmt werden und die Molekulargewichtsverteilung (Mw/Mn) durch Gelpermeationschromatographie (GPC) bestimmt wird.

**7.** Verfahren zur Herstellung eines Spinnvliesstoffs nach Anspruch 6, wobei der Mischungsschritt ein Schritt des Erhaltens einer Zusammensetzung durch weiteres Mischen einer Fettsäureamid mit 15 bis 22 Kohlenstoffatomen ist und ein Gehalt der Fettsäureamid mit 15 bis 22 Kohlenstoffatomen von 0,1 Masse-% bis 5,0 Masse-% in Bezug auf eine Gesamtmenge der Zusammensetzung beträgt.

## Revendications

**1.** Tissu non-tissé filé-lié, comprenant :

une fibre formée d'une composition contenant :

un homopolymère de propylène ayant un point de fusion de 140 °C ou plus ;

un polyéthylène ; et

au moins un polymère sélectionné parmi le groupe constitué d'un polymère représenté en (I), et d'un polymère représenté en (II),

dans lequel la fibre possède une structure mer-îlots,

une longueur moyenne d'une phase îlot, telle que déterminée par le procédé indiqué dans le passage de la description intitulé « (1) Longueur moyenne de phase îlot [$\mu$m] », est de 1 $\mu$m à 100 $\mu$m dans une section transversale prise le long d'une direction d'axe majeur de la fibre,

une teneur du polyéthylène est de 1,0 % en masse à 15,0 % en masse par rapport à une quantité totale de la composition,

une teneur de l'au moins un polymère sélectionné parmi le groupe constitué du polymère représenté en (I) et du polymère représenté en (II) est de 5,0 % en masse à 30,0 % en masse par rapport à une quantité totale de la composition, et

une teneur de l'homopolymère de propylène ayant un point de fusion de 140 °C ou plus est de 55,0 % en masse à 90,0 % en masse par rapport à une quantité totale de la composition :

(I) un copolymère aléatoire d'un propylène avec au moins l'un sélectionné parmi éthylène ou $\alpha$-oléfine ayant de 4 à 20 atomes de carbone, et

(II) un homopolymère de propylène ayant un point de fusion de moins de 120 °C satisfaisant les (a) à (f) suivants :

(a) [mmmm] = 20 % en moles à 60 % en moles ;

(b)

$$[rrrr]/(1 - [mmmm]) \leq 0,1 ;$$

(c)

$$[rmrm] > 2,5 \%$$

en moles,

(d)

$$[mm] \times [rr]/[(mr)]^2 \leq 2,0 ;$$

(e)

$$\text{poids moléculaire moyen en poids (Mw) = de 10 000 à 200 000 ;}$$

et

(f)

$$\text{distribution de poids moléculaire (Mw/Mn) < 4,}$$

dans lequel, dans (a) à (d) :

[mmmm] est une fraction mésopentade ;

[rrrr] est une fraction pentade racémique ;

[rmrm] est une fraction méso racémique mésopentade racémique ; et

[mm], [rr] et [mr] sont respectivement des fractions triade de celles-ci, [mmmm], [rrrr], [rmrm], [mm], [rr] et [mr] étant déterminées conformément au procédé proposé dans A. Zambelli, et al., « Macromolecules, 6, 925 (1973) », et la distribution de poids moléculaire (Mw/Mn) étant déterminée par chromatographie par perméation de gel (GPC).

2. Tissu non-tissé filé-lié selon la revendication 1, dans lequel une densité du polyéthylène est de 0,941 g/cm$^3$ à 0,970

g/cm$^3$.

3. Tissu non-tissé filé-lié selon la revendication 1 ou la revendication 2, dans lequel la composition contient un amide d'acide gras ayant 15 à 22 atomes de carbone, et une teneur de l'amide d'acide gras ayant 15 à 22 atomes de carbone est de 0,1 % en masse à 5,0 % en masse par rapport à une quantité totale de la composition.

4. Tissu non-tissé filé-lié selon l'une quelconque des revendications 1 à 3, dans lequel le polymère représenté en (I) est un copolymère aléatoire contenant au moins une unité constitutive dérivée de propylène et une unité constitutive dérivée d'éthylène.

5. Matériau hygiénique comprenant le tissu non-tissé filé-lié selon l'une quelconque des revendications 1 à 4.

6. Procédé de production d'un tissu non-tissé filé-lié selon la revendication 1, le procédé comprenant :

une étape de filtration dans laquelle du polyéthylène est fondu à une température de 180 °C à 200 °C, et le polyéthylène fondu est passé à travers un tamis ayant une ouverture de 65 $\mu$m ou moins ;
une étape de mélange dans laquelle le polyéthylène qui est passé à travers le tamis à l'étape de filtration, un homopolymère de propylène ayant un point de fusion de 140 °C ou plus, et au moins un polymère sélectionné parmi le groupe constitué d'un polymère représenté en (I), et d'un polymère représenté en (II) sont mélangés pour obtenir une composition ; et
une étape de formation de tissu non-tissé dans laquelle un tissu non-tissé est obtenu, par un procédé de filage-liage, à partir de la composition obtenue à l'étape de mélange,
dans lequel un nombre de particules ayant chacune un diamètre de 0,2 mm ou moins contenues dans un film formé du polyéthylène qui est passé à travers le tamis à l'étape de filtration est de 25 éléments/g ou moins,
une teneur du polyéthylène utilisé à l'étape de mélange est de 1,0 % en masse à 15,0 % en masse par rapport à une quantité totale de la composition,
une teneur de l'au moins un polymère sélectionné parmi le groupe constitué du polymère représenté en (I) et du polymère représenté en (II) utilisé à l'étape de mélange est de 5,0 % en masse à 30,0 % en masse par rapport à une quantité totale de la composition, et
une teneur de l'homopolymère de propylène ayant un point de fusion de 140 °C ou plus utilisé à l'étape de mélange est de 55,0 % en masse à 90,0 % en masse par rapport à une quantité totale de la composition :

(I) un copolymère aléatoire d'un propylène avec au moins l'un sélectionné parmi éthylène ou $\alpha$-oléfine ayant de 4 à 20 atomes de carbone, et
(II) un homopolymère de propylène ayant un point de fusion de moins de 120 °C satisfaisant les (a) à (f) suivants :

(a) [mmmm] = 20 % en moles à 60 % en moles ;
(b)

$$[rrrr]/(1 - [mmmm]) \leq 0,1 \ ;$$

(c)

$$[rmrm] > 2,5 \ \%$$

en moles,
(d)

$$[mm] \times [rr]/[(mr)]^2 \leq 2,0 \ ;$$

(e)

$$\text{poids moléculaire moyen en poids (Mw)} = \text{de } 10\ 000 \text{ à } 200\ 000 \ ;$$

et

(f)

## distribution de poids moléculaire (Mw/Mn) < 4,

dans lequel, dans (a) à (d) :

[mmmm] est une fraction mésopentade ;
[rrrr] est une fraction pentade racémique ;
[rmrm] est une fraction méso racémique mésopentade racémique ; et
[mm], [rr] et [mr] sont respectivement des fractions triade de celles-ci, [mmmm], [rrrr], [rmrm], [mm], [rr] et [mr] étant déterminées conformément au procédé proposé dans A. Zambelli, et al., « Macromolecules, 6, 925 (1973) », et la distribution de poids moléculaire (Mw/Mn) étant déterminée par chromatographie par perméation de gel (GPC).

7. Procédé de production d'un tissu non-tissé filé-lié selon la revendication 6, dans lequel l'étape de mélange est une étape consistant à obtenir une composition en mélangeant en outre un amide d'acide gras ayant 15 à 22 atomes de carbone, et une teneur de l'amide d'acide gras ayant 15 à 22 atomes de carbone est de 0,1 % en masse à 5,0 % en masse par rapport à une quantité totale de la composition.

FIG.1A

FIG.1B

FIG.2

FIG.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H09512313 A
- WO 2014050965 A
- JP 2015071854 A
- WO 2017006972 A
- WO 2003087172 A

**Non-patent literature cited in the description**

- **A. ZAMBELLI**. *Macromolecules*, 1973, vol. 6, 925
- **A. ZAMBELLI et al.** *Macromolecules*, 1973, vol. 6, 925
- **A. ZAMBELLI et al.** *Macromolecules*, 1975, vol. 8, 687